# EUROPEAN PATENT APPLICATION

(11) **EP 4 751 645 A1**
(43) Date of publication of application: **03.06.2026**
(21) Application number: 24306993.7
(22) Date of filing: 28.11.2024
(51) Int. Cl.: A61B 5/053, A61B 5/291, A61B 5/30, A61B 5/0531, A61B 5/0537, A61B 5/296, A61B 5/00

(54) **PREAMPLIFIER FOR BIOSIGNAL RECORDER**

(71) Applicant: Bioserenity Medical Devices Group, 75013 Paris (FR)
(72) Inventor: PROT, Pierre, 75013 Paris (FR)
(74) Representative: Icosa

(57) **Abstract**

The present invention relates to a preamplifier (10) configured to provide signals (52) to a recorder (20) of the biopotential;
the preamplifier (10) comprising:
- a first channel (14) connected to the input (11), comprising an amplifier (15) presenting a predefined gain (53) and configured to generate an amplified signal (54) via the multiplication of the non-amplified signal (51) by the gain (53);
- a first output (12) connected to the amplifier (15), and configured to be removably plugged to the recorder (20) in order to provide the amplified signal (54) to the recorder (20);

wherein:
- the preamplifier (10) is comprised in a housing separate from the recorder (20); and
- the preamplifier (10) comprises a second channel (16) configured to be connected to the electrode (31) and to provide the non-amplified signal (51) to the recorder (20), without amplification.

## Description

### FIELD OF INVENTION

The present invention relates to devices intended to record biosignals of living beings, such as electroencephalograph recorders for humans.

### BACKGROUND OF INVENTION

Devices for recording biosignals of living beings are know from the prior art.

When the signal to record has a low level, such as brainwaves, it may be necessary to amplify the signal before recording it.

Some preamplifiers are implemented internally to electroencephalogram ("EEG") recorders. Preamplified electrodes, which amplify the signal as proximate to the skin it can be, also exist. But even if the preamplification allows to detect signals of low levels, these devices are not always well suited for cerebral death diagnosis: indeed, when investigating cerebral death, a physician needs to be certain that there is no cerebral activity at all. The signal levels to detect are as low as a few microvolts.

Moreover, the quality of the signal needs to be certain: if an electrode, used to detect the brain activity, is poorly disposed on the skin, electric disturbances of the signal, or "noise" may result of the poor setup. The noise will be amplified by the preamplifier and can be interpreted as a brain activity.

The impedance of contact between the electrode and the skin can be measured in order to verify the correct setup of the electrode. Nevertheless, the preamplified electrodes prevent to conduct a such measure by the EEG recorder because the preamplifier inserts between the recorder and the electrode.

By consequence, there is a difficulty to obtain a trustworthy signal for brain death investigation, especially with systems that feature a signal preamplifier that is external to the EEG recorder.

An EEG recording system which has not been specifically designed for brain death investigation cannot be used for this kind of application, even if it comprises a contact impedance measuring device: its intrinsic noise level may not be suited for brain death investigation.

### SUMMARY

The purpose of the invention is to propose a device which renders an existing biosignals recording device compatible with very low levels signal investigation.

For that, it is proposed a preamplifier configured to be linked to at least one electrode being applied to a skin of a patient in order to provide a measure of a biopotential of the patient, the preamplifier being configured to exchange signals with a recorder of the biopotential;
the preamplifier comprising:
- an input, configured to be connected to the at least one electrode,
- a first channel connectable to the input, comprising an amplifier presenting a predefined gain and configured to generate an amplified signal via the multiplication of the measure by the gain;
- a first output connectable to the first channel, and configured to be removably plugged to the recorder in order to provide the amplified signal to the recorder.

According to the invention:
- the preamplifier is comprised in a housing separate from the recorder; and
- the preamplifier comprises a second channel configured to link the electrode to the recorder without amplification or with an amplification at least inferior to the gain, so that the recorder can provide a non-amplified signal to the electrode.

In that way, the recorder can exchange signals with the electrode through two separate channels:
- the first channel can be dedicated to the amplification of the measure in order to record low-level signals at a higher-level, raising them above an intrinsic noise of the recorder, and thus enhancing the signal-to-noise ratio ("SNR");
- the second channel can be dedicated to the verification of the impedance of contact between the electrode and the skin, without the amplification stage which would prevent this measure to be done. By consequence, the correct placement of the electrode on the skin can be verified, in order to assess that there will be no abnormal noise, that is to say a noise which level is too important in regard of a normal noise. An abnormal noise results from a poor electrode placement, that will pollute the quality of the signal before its amplification.

It is reminded that a voltage reference is required for the tension measuring of the electrodes. This reference can be:
- a reference electrode;
- a ground connection or a bias connection (which are also used to put the skin and the recorder to an equipotential;
- a mean of the values of several electrodes measuring, known as an "average reference". The biosignal value is thus measured by comparison with the voltage reference.

It is reminded that the measuring of the impedance is conducted as follows:
- the recorder sends a measuring electrical signal, of a known intensity, through the second output and towards the electrode, so that the signal reaches the skin,
- this signal intensity generates a voltage across the contact impedance between the electrode and the skin, before coming back to the recorder through the ground connection or the bias connection,
- the recorder measures the voltage of the measuring electrical signal, as it is received through the second channel,
- by dividing the voltage by the intensity, the impedance of the contact between the electrode and the skin is obtained.

If the EEG recording system comprised only amplified channels, the verification of the skin contact impedance could not have been verified. On the contrary, if the EEG recording system comprised only non-amplified channels, the brain death investigation could not have been conducted because of too high intrinsic noise of the recorder. The specific cooperation of the two channels allows to reach the purpose of the invention.

If needed, the values of the impedance can be registered by the recorder, which is helpful for conducting an investigation during several hours, without a constant surveillance by a physician. The validity of the investigation can be verified later. Generally, the impedance of contact is verified upon the placement of the electrodes, and is not necessarily verified later: registering the impedance values is not mandatory.

The amplified signal being provided at a higher level (and thus at a higher SNR) to the EEG recorder, while the impedance measurement capacity of the EEG recorder being preserved, diagnosis, such as brain death investigation, can therefore be conducted.

By "non amplified signal", we mean that the gain of the amplifier on the first channel is far greater than any gain which may be applied to a signal on the second channel. In an embodiment, the ratio of the valued between the amplified signal and the measure is greater than 5, or greater than 10, or greater than 20, or even greater than 30.

The preamplifier being autonomous, and being comprised inside a housing, it is possible to adapt an existing recorder device for the measure of low-level signals. For example, it is possible to render a generic existing EEG recorder, already having an impedance measurement device, compatible for the specific applications of cerebral death investigation, even if the EEG recorder does not feature noise performance that are sufficient for that application.

In order to provide the recorder with a non-amplified signal being as close as possible to the measure, the second channel comprises, at most, passive components or protective components, as a resistor or an inductance. That way, the preamplifier lets flow the current generated for the skin contact impedance measurement, which is generated by the EEG recorder, therefor impedance measurement capability of the recorder is preserved to allow the verification of the good contact between the electrode and the skin. Preferably, the second channel is even void of passive components, that means that the measure itself is directly provided to the recorder, without any transformation. In this embodiment, the non-amplified signal is the exact version of the measure.

In order to simplify the operation of the preamplifier, the second channel is connected to a second output of the preamplifier, configured to be removably plugged to another input port of the EEG recorder. This embodiment doubles the number of outputs needed for each electrode, but allows the simultaneous recording of the amplified signal, and the measure of the impedance.

In certain embodiments, and in order to choose which of the first channel and the second channel must be used, the preamplifier comprises a two-way selector, configured to link the input either to the amplifier or to the second channel. Alternatively, the preamplifier comprises a two-way selector, configured to either connect either the first channel or the second channel to the first output of the preamplifier.

The invention also relates to a system for recording a biopotential of a patient, comprising a preamplifier according to the preceding technical features, at least one electrode and a recorder.

Preferably, a first cable, which is configured to link the preamplifier to the at least one electrode, is shorter than a second cable, which is configured to link the preamplifier the recorder. This allows to reduce the distance between the electrode and the preamplifier, in order to reduce the exposition of the first cable to electromagnetic pollution prior to the amplification of the measure. Optionally, the preamp can be integrated directly into the active electrode.

Alternatively, in order to reduce the number of loose cables of the system, the preamplifier is configured to plug directly onto the recorder, connecting the first outputs of the preamplifier to inputs of the recorder without external cables.

When the preamplifier comprises two outputs for each electrode, and does not comprise a two-way selector between the amplifier and the second channel, the recorder is advantageously configured to calculate an actual value of the gain, by dividing the amplitude of amplified signal by the amplitude of the measure. This allows to further verify the relevancy of the signals provided to the recorder.

Further, the recorder is configured to verify a gain accuracy of the amplifier by comparing the actual value of the gain to an expected value of the gain. The integrity of the preamplifier can be verified.

When a physician wishes to verify the contact impedance, he can switch the functioning of the recorder so that it measures and displays the contact impedance to the physician. When the physician has finished his investigation, he can switch the functioning of the recorder back to registering the signals.

In order to assist a physician during an investigation with a biosignals, the recorder is configured to enable the execution of the following steps:
a) measure a contact impedance between the at least one electrode and the skin, then
b) if the contact impedance is above a predetermined threshold, emit an alert or display an information for a practitioner or interrupt the recording, or
c) if the contact impedance is above a predetermined threshold, record the amplified signal with the recorder.

The recorder can further be configured to execute the following steps:
a) measure the actual value of the gain, then
b) if the actual value of the gain is significantly different from the expected value of the gain, emit an alert for a practitioner, or stop the recording, or
c) if the actual value of the gain is not significantly different from the expected value of the gain (that is to say is sufficiently similar to the expected value of the gain), record the amplified signal with the recorder. Values exceeding the expected value of the gain by e.g. 2% or by 5% or more can be considered to be "significantly different".

The above process of amplifier gain check prevents from recording signals with a degraded hardware. In the context of brain death, a default in the amplifier that would lead to a very low gain would lead to a very low amplified signal. This could be interpretated as a false diagnosis of brain death, which is a critical risk for the patient.

### BRIEF DESCRIPTION OF THE DRAWINGS

**Figure** 1 is a schematic of a preferred embodiment of the invention, wherein the preamplifier comprises two outputs per electrode and is void of two-way selectors, a second channel of the preamplifier being connected to an input of the EEG recorder.
**Figure** 2 is a schematic of a second embodiment of the invention, wherein the preamplifier comprises two outputs per electrode, and a two-way selector between the amplifier and the second channel.
**Figure** 3 is a schematic of a third embodiment of the invention, wherein the preamplifier comprises a single output per electrode, and two-way selectors configured to connect either the first channel or the second channel to the single output.

### DETAILED DESCRIPTION

This invention relates to a device configured to allow a preexisting biosignals recorder (20) to access both an amplified signal (54) compatible with low-level biosignals investigation of a living being, and a non-amplified channel intended to measure an impedance of contact of an electrode placed on the skin of the living being, e.g. a person (40).

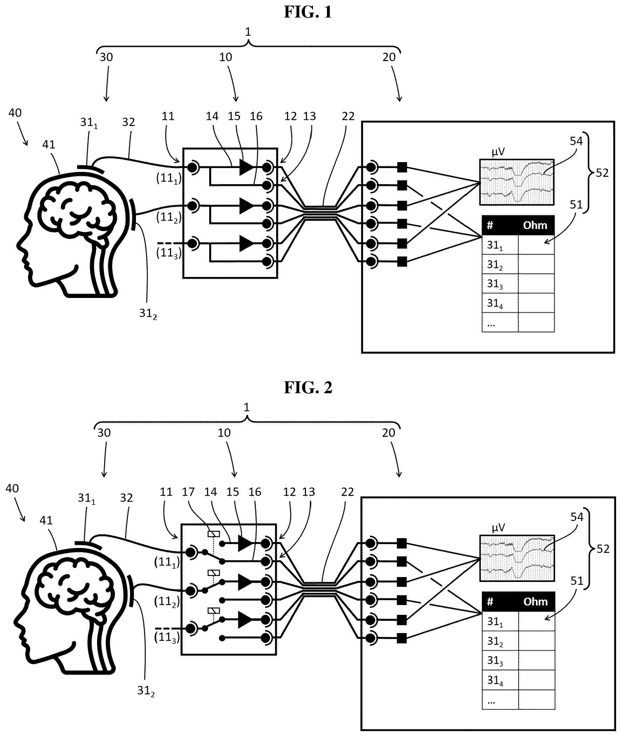

Figure 1 illustrates a preferred embodiment of the invention, wherein the biosignal is the brain activity of the person (40), and the recorder (20) is more specifically an electroencephalogram ("EEG") recorder (20).

Only EEG applications are illustrated, but the invention is compatible with any kind of biosignals analysis which may require the registering of low-level signals. For example, electro-gastrogram (EGG), electro-hysterogram (EHG), foetal electrocardiogram (fECG), are other examples of biosignals.

Only a few electrodes (31) are illustrated for the clarity of the drawings, but as many electrodes (31) as required can be used in the set of electrodes (31). At least two electrodes (31) are generally required, for measuring the difference of potential between the electrodes (31). One of these electrodes can serve as reference for measurements. In the preferred embodiment, this electrode will not be amplified and is directly connected to the reference input of the EEG recorder.

A first cable (32) links the electrode (31) to the input (11) of the preamplifier (10). The electrode (31) being generally disposable, the first input (11) is preferably a plug allowing to removably connect the electrode (31) to the preamplifier (10). Nevertheless, the preamplifier can also be integrated directly into the electrode. In certain embodiments, the electrodes are not removably pluggable ton the amplifier, but directly built in with the preamplifier.

In the preferred embodiment, each electrode (31) is connected to two outputs:
- a first output (12) is linked to the input (11) through a first channel (14) which comprises an amplifier (15); and
- a second output (13) linked to the input (11) through a second channel (16).

The purpose of the invention is to provide both an access to an amplified signal (54) for the recorder (20) to measure the biosignal, and a channel for a non-amplified signal (51) for the recorder (20) to measure the impedance of contact. By consequence:
- the second channel (16) can be void of any component susceptible of altering the measure, as illustrated on figure 1;
- the second channel (16) can comprise only passive components, that is to say components which will not modify significantly the signal in both direction between the input (11) and the second output (13). These passive components can be, for example, electromagnetic interference or electro-static discharge protection devices.

This kind of components can be as follows:
- either passive components, as a resistor, an inductance or a capacitor;
- either a reversed amplifier (15), in the meaning that it can amplify a signal going from the second output (13) in the direction of the input (11), but not a signal going from the input (11) in direction of the second output (13).

These components will not add noise, nor modify the non-amplified non-amplified signal (51) in a sufficient manner so that the recorder (20) could not assess the correct placement of the electrode (31) on the skin (41).

The amplifier (15) has a gain, and multiplies the value of the measure by the gain. For example, the level of the measure being as low as a few microvolts in the field of a brain death investigation, the gain needs to be sufficient to raise the amplified signal (54) higher than the intrinsic noise level of the EEG recorder (20). Gains of 10 to 20 will be usually chosen, but they can extend from 2 or 3 (if there is risk of saturation of the input) to 100, 1000 or even higher (if the recorder is not dedicated to EEG recording, and may be a noisier recorder). It can be technically difficult to implement high gain values, so the amplifier (15) can be split in a two-stages amplifier.

A second cable (22) links the preamplifier (10) to the recorder (20). As the preamplifier (10) of the invention is intended to upgrade a preexisting recorder (20), the second cable (22) preferably comprises plugs configured to be removably plugged, as this kind of connection is common on recorders (20). Advantageously, the second cable (22) is removably pluggable on the second output (13) of the preamplifier (10). In a specific embodiment, the preamplifier (10) can also be mechanically configured to directly fit with the recorder (20) so that no cable (22) is needed and the outputs (12) and (13) directly inserted in the recorder inputs. This embodiment reduces the number of cables required, providing less tangling during use, better usability, and potentially lower cabling costs.

The length of the first cable (32) has impacts on the immunity of the system to electromagnetic waves generated by surrounding electronics. The longer the cable (32), the more electromagnetic waves will be caught by the first cable (32) acting as an antenna, thus generating more noise in the signals conveyed by the first cable (32).

In order to reduce the noise resulting from electromagnetic pollution before the amplification of the measure, it is better to reduce the distance between the amplifier (15) and the electrode (31). if a second cable (22) is present, the first cable (32) is preferably shorter than the second cable (22). The first cable (32) has preferably a length inferior to 150cm, or even preferably inferior to 50cm.

The signals (52) used by the recorder (20) comprise both the amplified signal (54), and a non-amplified signal (51) obtained from the electrode (31).

The amplified signal (54) is used in the same manner as for a common recording over time, for example an EEG.

The non-amplified signal (51) is used to verify the good connexion between the electrode (31) and the skin (41) of the patient (40).

The verification of the good connexion is conducted with a measure of the impedance of the contact between the electrode (31) and the skin (41).

The measuring of the impedance is conducted as follows:
- the recorder (20) sends a measuring electrical signal, of a known intensity, through the second output (13) and towards the electrode (31), so that the signal reaches the skin (41),
- the recorder (20) measures the tension of the measuring electrical signal, being amplified as it is received through the second channel,
- by dividing the tension by the intensity, the impedance of the contact between the electrode (31) and the skin (41) is obtained.

A limited impedance means that the electrode (31) is correctly placed on the skin (41). This is a criteria of good signal quality, which is required to be checked before recording the biosignal over time.

On the contrary, a too high impedance means the electrode (31) is not in good contact with the skin (41), and the signal may be impaired with interferences and noise. When recorded, this noise could lead to impossibility for the physician to put a diagnosis, or even to put a wrong diagnosis.

In order to automatise the verification of the impedance, the recorder (20) can be configured to automatically execute the following steps:
a) measure a contact impedance between the at least one electrode (31) and the skin (41), then
b) if the contact impedance is above a predetermined threshold, emit an alert for a practitioner, or interrupt the registering, or
c) if the contact impedance is above a predetermined threshold, record the amplified signal (54) with the recorder (20).

Eventually, the impedance can be continuously monitored while recording the signal. In that case, an alert can be issued. The alert can be visual, or auditive. The alert can also be warning superimposed to the actual recording.

This verification of the impedance does not need to be continuous. A discrete verification, for example once every hour, can be sufficient.

In order to measure the impedance continuously without interrupting the recording of the amplified signal (54), the recorder (20) is configured to conduct a multiplexing over frequency of the signals: the measuring signal and the recording signal present different frequencies, so they can be conveyed over the same network.

The frequency multiplexing can also be used to differentiate the simultaneous measures of different electrodes (31) used.

The values of the impedances can be stored in a database, or superimposed on the registering, or displayed on a screen of the recorder (20).

Advantageously, the recorder (20) is configured to execute the following steps:
a) measure the actual value of the gain, then
b) if the actual value of the gain is significantly different from the expected value of the gain, emit an alert for a practitioner, or interrupt the registering, or
c) if the actual value of the gain is sufficiently similar to the expected value of the gain, record the amplified signal (54) with the recorder (20).

The acceptable level of difference between the expected gain value and the actual gain value can be defined by a threshold, e.g. 5% or 10%.

Again, the alert can be visual, auditive, or be a warning superimposed to the actual recording.

The verification of the gain is typically conducted during the setup of the system, prior to a recording. The verification of the gain can also be conducted during a maintenance routine of the preamplifier (10). If required, the gain can be monitored continuously.

The knowledge of the exact value of the gain can be useful for critical investigations, such as brain death investigations: if the actual value of the gain is too low, then the recording will show signals of poor levels and the physician may not take the appropriate decision.

In reference to figure 2, a second embodiment of the invention differs in that the first channel (14) and the second channel (16) are not connected together with the input (11): a two-way selector (17), e.g. a switch or a relay, can select which one of the first channel (14) and the second channel (16) is connected to the input (11).

During the recording, the first channel (14) is connected to the input (11).

During the measure of the impedance, the second channel (16) is connected to the input (11).

The two-way selectors (17) can be automatically driven by a program of the recorder.

On the drawing of figure 2:
- a first electrode (31₁) is connected to its second channel (16), and is configured to verify the impedance of contact with the skin (41);
- a second electrode (31₂) and a third electrode (31₃) are connected to their first channel (14) so that the recorder (20) can receive the amplified signals (54).

In this embodiment, it is not possible to measure the impedance during a recording, as one electrode (31) cannot be simultaneously connected to its first channel (14) and its second channel (16).

Nevertheless, this embodiment presents a different layout which can be advantageous according to the preamplifier (10) design.

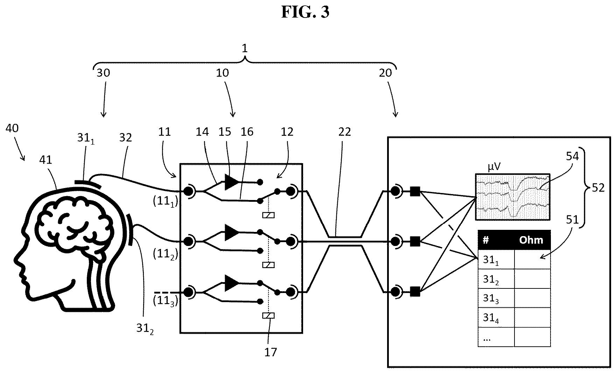

In reference to the figure 3, a third embodiment is illustrated. This embodiment is advantageous as it requires a single output (11) per electrode (31), whereas the previous embodiments required two outputs (11, 13) per electrode (31).

Indeed, each output (11) is connected both to the first channel (14) and to the second channel (16).

Two-way selectors (17) are used to either disconnect the second channel (16) from the electrode (31), either connect the second channel (16) to the electrode (31).

When the second channel (16) is disconnected, the electrode (31) can be used in a measuring configuration.

When the second channel (16) is connected, the first channel (14) is by-passed and the electrode (31) can be used for measuring the impedance of contact between the skin (41) and the electrode (31).

The measuring of impedance is conducted in a same manner as for the second embodiment.

As for the second embodiment, the third embodiment is not compatible with impedance verification during a recording. Moreover, it requires switch control by the recorder (20), which implies a digital control link from the recorder (20) to the preamplifier (10). This solution can only be practicable to specific EEG recorders whose hardware and firmware are specifically designed to control the preamplifier (10) switches (17) as required.

Nevertheless, the third embodiment has the advantage of being more compact, as less outputs (11) are required. Moreover, the wiring inside the preamplifier (10) is reduced, further aiming to have a compact preamplifier (10).

## Claims

1. Preamplifier (10) configured to be linked to at least one electrode (31) being applied to a skin (41) of a patient (40) or an animal in order to provide a measure of a biopotential of the patient (40), the preamplifier (10) being configured to exchange signals (52) with a recorder (20) of the biopotential;
the preamplifier (10) comprising:
- an input (11), configured to be connected to the at least one electrode (31),
- a first channel (14) connectable to the input (11), comprising an amplifier (15) presenting a predefined gain (53) and configured to generate an amplified signal (54) via the multiplication of the measure by the gain (53);
- a first output (12) connectable to the first channel (14), and configured to be removably plugged to the recorder (20) in order to provide the amplified signal (54) to the recorder (20);
**characterized in that**:
- the preamplifier (10) is comprised in a housing separate from the recorder (20); and
- the preamplifier (10) comprises a second channel (16) configured to link the electrode (31) to the recorder (20) without amplification or with an amplification at least inferior to the gain (53), so that the recorder (20) can provide a non-amplified signal (51) to the electrode (31).

2. Preamplifier (10) according to claim 1, wherein the second channel (16) comprises at most passive or protective components so as to preserve the non-amplified signal (51), preferably the second channel (16) is void of passive components.

3. Preamplifier (10) according to claim 1 or 2, wherein the second channel (16) is connected to a second output (13) of the preamplifier (10), configured to be removably plugged to the recorder (20).

4. Preamplifier (10) according to claim 2 or 3, wherein the preamplifier comprises a two-way selector (17), configured to connect either the first channel (14), or the second channel (16), to the first output (12) of the preamplifier (10).

5. System (1) for recording a biopotential of a patient (40), comprising a preamplifier (10) according to one of the claims 1 to 5, at least one electrode (31) and a recorder (20).

6. System (1) according to claim 5, wherein a first cable (32) configured to link the preamplifier (10) to the at least one electrode (31) is shorter than a second cable (22) configured to link the preamplifier (10) the recorder (20).

7. System (1) according to claim 5, wherein the preamplifier (10) is configured to plug directly onto the recorder (20), connecting the first outputs (12) of the preamplifier (10) to inputs of the recorder (20) without external cables.

8. System (1) according to one of the claims 5 to 7, comprising a preamplifier according to claim 3, wherein the recorder (20) is configured to calculate an actual value of the gain (53), by dividing the amplitude of amplified signal (51) by the amplitude of the measure (51).

9. System (1) according to claim 8, wherein the recorder (20) is configured to verify a gain (53) accuracy of the amplifier (15) by comparing the actual value of the gain (53) to an expected value of the gain (53).

10. System (1) according to claim 9, wherein the recorder (20) is configured to enable the execution of the following steps:
a) measure a contact impedance between the at least one electrode (31) and the skin (41), then
b) if the contact impedance is above a predetermined threshold, emit an alert or display an information for a practitioner, or
c) if the contact impedance is below a predetermined threshold, record the amplified signal (54) with the recorder (20).

11. System (1) according to claim 10, wherein the recorder (20) is further configured to execute the following steps:
a) measure the actual value of the gain (53), then
b) if the actual value of the gain (53) significantly different from the expected value of the gain (53), emit an alert for a practitioner, or
c) if the actual value of the gain (53) is sufficiently similar to the expected value of the gain (53), record the amplified signal (54) with the recorder (20).
